# EUROPÄISCHE PATENTSCHRIFT

(11) **EP 1 951 904 B1**
(45) Veröffentlichungstag und Bekanntmachung des Hinweises auf die Patenterteilung: **12.01.2011**
(21) Anmeldenummer: 06829945.2
(22) Anmeldetag: 06.11.2006
(51) Int. Cl.: C12Q 1/68

(54) **VORRICHTUNG UND VERFAHREN ZUR AUTOMATISIERTEN ISOLIERUNG UND AUFREINIGUNG VON NUKLEINSÄUREN AUS BELIEBIGEN KOMPLEXEN AUSGANGSMATERIALIEN**
DEVICE AND METHOD FOR THE AUTOMATIC ISOLATION AND PURIFICATION OF NUCLEIC ACIDS FROM ANY COMPLEX STARTING MATERIALS
PROCEDE ET DISPOSITIF POUR ISOLER ET purifier AUTOMATIQUEMENT DES ACIDES NUCLEIQUES A PARTIR DE MATIERES PREMIERES EVENTUELLEMENT COMPLEXES

(30) Priorität: 06.11.2005 DE 102005053463
(43) Veröffentlichungstag der Anmeldung: 06.08.2008
(73) Patentinhaber: AJ Innuscreen GmbH, 13125 Berlin (DE); AJ Cybertron Gesellschaft für Laborautomationssysteme mbH, 12487 Berlin (DE)
(72) Erfinder: TIMO, HILLEBRAND, 15366 Hoenow (DE); MATTHIAS, ARNDT, 13591 Berlin (DE); UWE, WELLNITZ, 13125 Berlin (DE); KLAUS, BERKA, 07743 Jena (DE); VOLKER, HILLEBRAND, 06862 Rosslau/Elbe (DE)
(74) Vertreter: Wehlan, Helmut
(86) Internationale Anmeldenummer: PCT/EP2006/068147
(87) Internationale Veröffentlichungsnummer: WO 2007/051859

(56) Entgegenhaltungen:
- EP-A2- 0 941 766
- WO-A-96/41810
- WO-A-2005/065831
- DE-A1- 19 834 584
- HOURFAR M K ET AL: "High-throughput purification of viral RNA based on novel aqueous chemistry for nucleic acid isolation" CLINICAL CHEMISTRY, AMERICAN ASSOCIATION FOR CLINICAL CHEMISTRY, WASHINGTON, DC, US, Bd. 51, Nr. 7, Juli 2005 (2005-07), Seiten 1217-1222, XP002403248 ISSN: 0009-9147

## Beschreibung

Gegenstand der Erfindung ist eine Vorrichtung und ein Verfahren zur automatisierten Isolierung von Nukleinsäuren aus nukleinsäurehaltigen unterschiedlichsten Ausgangsmaterialien, welches sowohl eine sehr hohe Qualität der zu isolierenden Nukleinsäuren garantiert als auch die Isolierung quantitativer Ausbeuten ermöglicht.

Unter klassischen Bedingungen erfolgt die Isolierung von DNA aus Zellen und Geweben dadurch, dass die Nukleinsäuren enthaltenden Ausgangsmaterialien unter stark denaturierenden und reduzierenden Bedingungen, teilweise auch unter Verwendung von proteinabbauenden Enzymen, aufgeschlossen, die austretenden Nukleinsäurefraktionen über Phenol-/Chloroform-Extraktionsschritte gereinigt und die Nukleinsäuren mittels Dialyse oder Ethanolpräzipitation aus der wässrigen Phase gewonnen werden (Sambrook, J., Fritsch, E.F. und Maniatis, T., 1989, CSH, "Molecular Cloning").

Diese "klassischen Verfahren" zur Isolierung von Nukleinsäuren aus Zellen und besonders aus Geweben sind sehr zeitaufwendig (teilweise länger als 48 h), erfordern einen erheblichen apparativen Aufwand und sind darüber hinaus auch nicht unter Feldbedingungen realisierbar. Außerdem sind solche Methoden auf Grund der verwendeten Chemikalien wie Phenol und Chloroform in einem nicht geringen Maße gesundheitsgefährdend.

Verschiedene alternative Verfahren zur Isolierung von Nukleinsäuren aus unterschiedlichen biologischen Ausgangsmaterialien ermöglichen die aufwendige und gesundheitsschädigende Phenol-/Chloroform-Extraktion von Nukleinsäuren zu umgehen, sowie eine Reduzierung der zeitlichen Aufwendungen zu erreichen.

Alle diese Verfahren basieren auf einer von Vogelstein und Gillespie (Proc. Natl. Acad. Sci. USA, 1979, 76, 615-619) entwickelten und erstmals beschriebenen Methode zur präparativen und analytischen Reinigung von DNA-Fragmenten aus Agarosegelen. Die Methode kombiniert die Auflösung der die zu isolierende DNA- Bande enthaltende Agarose in einer gesättigten Lösung eines chaotropen Salzes (NaJ) mit einer Bindung der DNA an Glaspartikel. Die an die Glaspartikel fixierte DNA wird anschließend mit einer Waschlösung (20 mM Tris HCl [pH 7,2]; 200mM NaCl; 2 mM EDTA; 50% v/v Ethanol) gewaschen und danach von den Trägerpartikeln abgelöst.

Diese Methode erfuhr bis heute eine Reihe von Modifikationen und wird zum gegenwärtigen Zeitpunkt für unterschiedliche Verfahren der Extraktion und Reinigung von Nukleinsäuren aus unterschiedlichen Herkünften angewendet (Marko, M.A., Chipperfield, R. und Bimboim, H.G., 1982, Anal. Biochem., 121, 382-387).

Darüber hinaus existieren heute weltweit auch eine Vielzahl von Reagenziensystemen vor allem zur Reinigung von DNA-Fragmenten aus Agarosegelen und für die Isolierung von Plasmid DNA aus bakteriellen Lysaten, aber auch für die Isolierung von längerkettigen Nukleinsäuren (genomische DNA, zelluläre Gesamt-RNA) aus Blut, Geweben oder auch Zellkulturen .

Alle diese kommerziell verfügbaren Kits basieren auf dem hinlänglich bekannten Prinzip der Bindung von Nukleinsäuren an mineralische Träger unter Anwesenheit von Lösungen unterschiedlicher chaotroper Salze und verwenden als Trägermaterialien Suspensionen feingemahlener Glaspulver (z.B. Glasmilk , BIO 101, La Jolla, CA), Diatomenerden (Fa. Sigma) oder auch Silicagele. (Diagen, DE 41 39 664 A1).

Ein für eine Vielzahl unterschiedlicher Anwendungen praktikables Verfahren zur Isolierung von Nukleinsäuren ist in US 5,234,809 (Boom) dargestellt. Dort ist ein Verfahren zur Isolierung von Nukleinsäuren aus nukleinsäurehaltigen Ausgangsmaterialien durch die Inkubation des Ausgangsmaterials mit einem chaotropen Puffer und einer DNA-bindenden festen Phase beschrieben. Die chaotropen Puffer realisieren sowohl die Lyse des Ausgangsmaterials als auch die Bindung der Nukleinsäuren an die feste Phase. Das Verfahren ist gut geeignet, um Nukleinsäuren aus kleinen Probenmengen zu isolieren und fmdet speziell im Bereich der Isolierung viraler Nukleinsäuren seine praktische Anwendung.

Spezifische Modifikationen dieser Verfahren betreffen den Einsatz von neuartigen Trägermaterialien, welche für bestimmte Fragestellungen applikative Vorteile zeigen (WO-A 95/34569).

In neueren Patentschriften wird offenbart, dass für die Adsorption von Nukleinsäuren an die dem Fachmann bekannten und eingesetzten silikatischen Materialien auch sogenannte antichaotrope Salze als Bestandteil von Lyse/Bindungspuffet-Systemen sehr effizient und erfolgreich eingesetzt werden können (EP 1135479).

Zum Stand der Technik gehört auch die US-Patentschrift 6,207,445 B1, in der eine einfach zu verwendende Filtrations- und Extraktionsvorrichtung für ein biologisches Fluid, die eine Probe direkt einem analytischen Verfahren zur Verfügung stellt, beschrieben wird. Die Vorrichtung ist im Stande, eine geklärte Flüssigkeit, die direkt für eine Analyse oder für eine Entsorgung, wie es für den spezifischen Analyt von Interesse geeignet ist, zur Verfügung zu stellen, und sie ist im Stande, teilchenförmige Materialien einzufangen und erlaubt eine weitere Verarbeitung, d. h. eine Extraktion dieser Teilchen direkt mit der Vorrichtung. Sobald sie einmal extrahiert ist, stellt die Vorrichtung eine Flüssigkeit, die den Analyten von Interesse enthält, einem analytischen Verfahren zur Verfügung. Die Vorrichtung schließt einen biegsamen Körper mit einem offenen oberen Ende und einer Innenwand, welche eine innere Kammer definiert, ein. Ein Verschluss- bzw. Versiegelungsmechanismus ist so angelegt, um dass offene obere Ende des Körpers zu versiegeln. Eine Gradienten-Filteranordnung, welche mindestens einen Filter einschließt, wird in dem Körper durch eine Trägeranordnung getragen. Der biegsame Körper ist so ausgelegt, um durch die Finger eines Benutzers zusammengedrückt zu werden, um in der Kammer einen positiven Druck aufzubauen, der ausreicht, um ein Fluid in der Kammer durch die Filteranordnung fließen zu lassen.

Im US-Patent 6,207,463 wird eine Vorrichtung zum Trennen magnetischer Partikel von einer Stoffzusammensetzung beschrieben. Diese Vorrichtung enthält eine langgestreckte Schutzhülle mit einem oberen und einem unteren Ende, einen von der Schutzhülle umschlossenen Innenraum, der sich von deren oberem zu ihrem unterem Ende erstreckt, einen verstellbaren Magnetstab, der in dem Innenraum, in dessen Längsrichtung gerichtet, angeordnet ist.

Eine Vorrichtung zum Trennen und Reinigen einer Suspension mit magnetischen Partikeln wird in der internationalen Patentanmeldung WO2005/063831 A1 beschrieben. Diese Vorrichtung umfasst eine Prozessareal mit getaktet wandernden Einrichtungen für den Transport der magnetischen Mikropartikel in x-Richtung.

Die Analyse des Standes der Technik verdeutlicht eindrucksvoll, dass eine Vielzahl von Möglichkeiten existiert, Nukleinsäuren an feste Trägermaterialien, insbesondere mineralische Trägermaterialien auf Siliziumbasis, zu binden, nachfolgend zu waschen und die Nukleinsäuren vom Trägermaterial wieder abzulösen.

Auf Basis der beschriebenen Technologien zur Anbindung von Nukleinsäuren an feste Trägermaterialien existiert eine Vielzahl von kommerziell verfügbaren Produkten, die es den Anwendern erlauben, Nukleinsäuren aus nukleinsäurehaltigen Ausgangsmaterialien zu isolieren. Sehr interessant sind dabei zunehmend Verfahrenslösungen, die es gestatten, Nukleinsäuren automatisiert zu isolieren und aufzureinigen. Dies liegt darin begründet, den erheblichen manuellen Aufwand zu reduzieren bzw. auch hohe Durchsätze an Nukleinsäure-Extraktionen realisieren zu können. Diesen Anforderungen Rechnung tragend, existieren roboter- bzw. automatenbasierte Lösungen zur Isolierung von Nukleinsäuren. Dabei handelt es sich i. d. R. um sehr aufwendige Gerätekonfigurationen, die eine Isolierung von Nukleinsäuren ermöglichen. Man kann generell zwei Systemkonfigurationen unterscheiden.
1. Automatisierte Verfahren zur Isolierung von Nukleinsäuren unter Nutzung von 96-Well-Filterplatten zur Anbindung der Nukleinsäuren.
2. Automatisierte Verfahren zur Isolierung von Nukleinsäuren unter Nutzung von magnetischen Partikeln zur Anbindung der Nukleinsäuren.

Der Prozess der Isolierung der Nukleinsäuren realisiert sich dabei in Analogie zu den beschriebenen manuellen Verfahren. Nach Lyse des Ausgangsmaterials erfolgt die Bindung der Nukleinsäuren an die jeweiligen festen Trägermaterialien (Filterplatte oder Magnetpartikel), nachfolgend das Waschen der gebundenen Nukleinsäure, Trocknen der Trägermaterialien (Entfernung alkoholischer Komponenten) und Elution der Nukleinsäuren. Dabei erfolgt die Prozessierung der Extraktion im Falle der Nutzung von Filterplatten via Vakuum oder mittels Druck bzw. bei Nutzung von magnetischen Partikeln über eine magnetische Separation.

Aufgabe der Roboterstationen ist es, wenn möglich, alle notwendigen Verfahrensschritte einer Nukleinsäureextraktion automatisiert abzubilden. Dies erfolgt dabei über aufwendige Pipettierschritte mittels Dispensiereinrichtungen, welche die unterschiedlichen Pufferkomponenten handhaben müssen, Vakuumstationen für die Filtration bzw. Magnetseparatoren für Nutzung magnetischer Partikel, ggf. Integration aufwendiger Zentrifugationstechnik. Notwendig sind auch Heizeinheiten und Schütteleinheiten zur Unterstützung von Lyseprozessen.

Allerdings existieren nur sehr wenige hochspezialisierte Automaten, die es erlauben, den gesamten Prozess der Isolierung von Nukleinsäuren durchzuführen. Darüber hinaus sind diese Geräte oftmals in keiner Weise universell einsetzbar, sondern immer nur optimiert für hochspezifische Applikationslösungen. Ein weiterer extremer Nachteil sind enorm hohe Kosten für die Anschaffung eines solchen Automaten. Dies wird noch verstärkt durch einen teilweise extrem hohen Einsatz von Verbrauchsmaterialien, insbesondere von Pipettenspitzen für die notwendigen Pipettierschritte. So werden bei bestimmten Anwendungen für die Isolierung von Nukleinsäuren mittels 96-Well-Filterpatten teilweise mehrere hundert Pipettenspitzen benötigt.

Preiswertere Systeme sind z.B. Gerätelösungen, die es erlauben, mittels magnetischer Partikel Nukleinsäuren teilautomatisiert zu isolieren. (z.B. KingFisher (Handelsnahme)). Diese Geräte sind speziell für die Isolierung von Nukleinsäuren entwickelt worden und arbeiten nach einem einfachen "Walk-Away"-Prinzip. Die zu bearbeitenden Proben sowie notwendige Pufferlösungen für die Schritte Binden- Waschen- Eluieren werden in Reaktionskavitäten überführt, wobei die Separation der magnetischen Partikel über Magnetstäbe erfolgt, welche Schritt für Schritt den Prozess der Nukleinsäureisolierung über die Kontaktierung der magnetischen Partikel mit den unterschiedlichen Pufferlösungen realisieren. Ein neueres Verfahren ist in der Patentschrift EP1382675 offenbart. Hierbei erfolgt der Prozess der Isolierung von Nukleinsäuren über die Verwendung einer neuartigen und extrem dünnen Membran. Diese Membran ist Bestandteil einer Filterkartusche. Diese Filtereinheit ist Kernstück eines Extraktionsgerätes, welches Teilprozesse der Isolierung von Nukleinsäuren (Binden-Waschen- Eluieren) automatisiert durchführt. Dabei wird vom Anwender eine zuvor lysierte Probe auf die Filtereinheit gegeben. Der Durchlauf der Probe über die Filtermembran kann über Vakuum erfolgen. Nachfolgende Waschlösungen werden automatisiert zudispensiert und über die Membran gesaugt. Der Eiutionsschritt erfolgt nach Zugabe eines Elutionspuffers in derselben Form.

Diese letzteren Gerätesysteme sind weitaus preiswerter als die schon beschriebenen Extraktionsroboter.

Nachteilig bei diesen Systemen ist dabei aber der extrem geringe Automatisierungsgrad. Diese Systeme automatisieren lediglich Teilschritte von Verfahren zur Isolierung von Nukleinsäuren. Der gesamte Vorgang der Probenvorbehandlung und insbesondere der Probenlyse erfolgt nicht mittels dieser Geräte. Die automatisierte Extraktion erfolgt erst nach der Lyse der Probe. Darüber hinaus wird nicht einmal die lysierte Probe automatisiert vom Gerät übernommen, sondern muss manuell zugegeben werden. Das System KingFisher bedarf des weiteren zeit- und arbeitsaufwendiger Schritte, um alle Pufferkomponenten in die dafür vorgesehene Reaktionsplastik zu füllen.

Der sehr geringe Automatisierungsgrad stellt damit eine große Limitierung des Einsatzgebietes dieser Geräte dar.

Ein weiteres wesentliches Problem im Zusammenhang mit einer Automatisierung von Verfahren zur Isolierung und Aufreinigung von Nukleinsäuren insbesondere aus komplexen Ausgangsmaterialien (Gewebe, Mausschwanz, Pflanzen, Lebensmittel, Stuhlproben etc.) besteht in der notwendigen Abtrennung von ungelösten Ausgangsmaterialien nach erfolgter Lyse. Dies kann bisher i.d.R. nur über einen Zentrifugationsschritt zur Pelletierung der ungelösten Komponenten (weiterarbeiten mit den Überständen) bzw. mittels einer Zentrifugation über einen Filter (weiterarbeiten mit den Filtraten) erreicht werden. Da die Integration solcher Verfahrenschritte auf die gängigen Automaten kaum möglich ist, ist die automatisierte Aufreinigung aus diesbezüglich problematischen Proben nicht möglich oder nur unzureichend realisiert.

Die Aufgabe der Erfindung bestand darin, die im Stand der Technik genannten Nachteile zu beseitigen.

Die Aufgabe wurde gemäß den Merkmalen der Patentansprüche gelöst.

Dabei wurden die notwendigen Prozesse der Isolierung von Nukleinsäuren hochautomatisiert realisiert, die Mengen an benötigten Verbrauchsmaterialien gering gehalten, flexible Probenumfänge bearbeitet und der Prozess der Isolierung der Nukleinsäuren beschleunigt.

Darüber wurden Nukleinsäuren aus schwierigen komplexen Proben, insbesondere in Hinblick auf das Vorliegen von festen ungelösten Komponenten, nach Lyse automatisiert und ohne zusätzliche manuelle Intervention isoliert. Außerdem wurde erstmals aus einer Probe simultan und parallel sowohl genomische DNA als auch zelluläre Total RNA isoliert.

Das erfindungsgemäße Verfahren und die erfindungsgemäßen Vorrichtungen erfüllen die gestellten Aufgaben in idealster Weise.

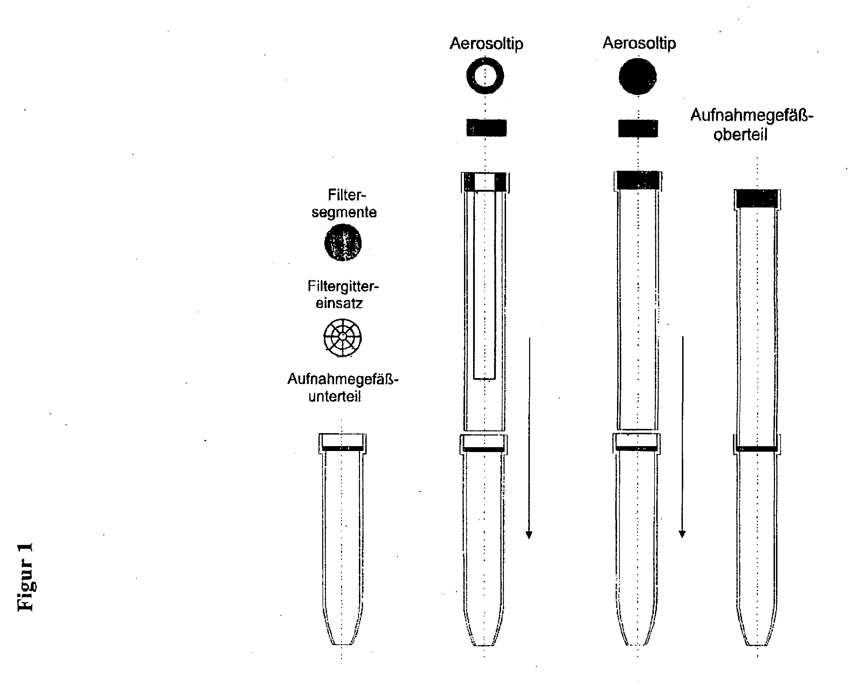

Kernstück des Extraktionsprozesses ist dabei eine Reaktionseinheit, welche sich aus einem Ober- und einem Unterteil zusammensetzt. Das Unterteil der Reaktionseinheit besteht dabei aus einer Reaktionskavität mit einem durchlässigen Filtergittereinsatz. Dieser Filtergittereinsatz dient im noch zu beschreibenden Verfahrensprozess des erfindungsgemäßen Verfahrens der Abtrennung von partikulären Komponenten nach Lyse einer komplexen biologischen Probe. Darüber hinaus dient dieser Einsatz optional auch als Auflagefläche für ein festes Trägermaterial für die Anbindung von zu isolierenden Biomolekülen (z.B. ein Glasfaservlies oder eine Membran). Diese Variante kommt insbesondere dann zum Einsatz, wenn aus einer biologischen Probe simultan die genomische DNA und die zelluläre Total RNA isoliert werden soll. Das Oberteil der Reaktionseinheit besteht aus einer Reaktionskavität, in welche eine Plastikhülse verbracht wird. Darüber hinaus enthält das Oberteil ein Filtermaterial zum Schutz vor Aerosolen. Die fertige Reaktionseinheit entsteht durch die Kombination von Oberteil und Unterteil. Wird ein chromatographisches Filtermaterial verwendet, dann wird dieses auch durch die Kontaktierung von Ober- und Unterteil auf dem Filtereinsatz fest fixiert. Dabei fixiert das Oberteil der Reaktionseinheit das Filtermaterial. Nach Kombination von Ober- und Unterteil ist die Reaktionseinheit funktionell aktiv. Die Figur 1 zeigt den Aufbau der erfindungsgemäßen Reaktionseinheit. Das erfindungsgemäße Mittel besitzt für den zu realisierenden Prozess der Isolierung von Nukleinsäuren eine Multiplexfunktion.

Das Mittel dient erfindungsgemäß:
1. einer exakten Dispensierung und Pipettierung von Flüssigkeiten,
2. einer Abtrennung von festen ungelösten Komponenten nach Lyse der zu bearbeitenden Probe,
3. der Isolierung von Nukleinsäuren unter Verwendung magnetischer Partikel,
4. der Isolierung von Nukleinsäuren über deren chromatographische Anbindung an ein festes Trägermaterial (als optionalem Bestandteil der Reaktionseinheit).

Die Reaktionseinheit ist dabei das Kernstück eines Automaten. Er besteht aus Positionen zur Aufnahme von handelsüblichen Reaktionsgefäßen sowie von Vorratsgefäßen für Flüssigkeiten sowie einer Steck-Position für die erfindungsgemäßen Reaktionseinheiten. Eine Reaktionsgefäß-Position ist potenziell beheizbar.

Der Extraktionsautomat enthält weiterhin eine Saug- bzw. Druckluftvorrichtung. Diese Einheit ist gekoppelt mit einem Dispensierkopf, welcher exakt auf die Reaktionseinheiten angepasst ist. Die Drucklufteinheit ist an eine Wärmequelle gekoppelt, die es ermöglicht, auch erwärmte Luft abzugeben. Im Dispensierkopf befindet sich weiterhin eine kinematische Einheit zur Vertikalbewegung von Magnetstiften. Darüber hinaus enthält der Extraktionsautomat eine Schnittstelle für die Kommunikation mit einem Rechner, welcher alle Programmabläufe steuert.

Ein erfindungsgemäßer Prozess der Isolierung von Nukleinsäuren, z.B. genomischer DNA aus einer biologischen Probe, wird mittels des erfindungsgemäßen Verfahrens wie folgt realisiert. Allgemein erfolgt die Bearbeitung einer Probe für die Isolierung und Aufreinigung von Nukleinsäuren nach dem allgemeinen Ablaufschema Lysieren- Binden- Waschen-Eluieren. Der Prozess kann dabei vollständig automatisiert erfolgen. Alle für die Extraktion benötigten Pufferkomponenten befinden sich auf dem Automaten in den Vorratsgefäßen bzw. in den Reaktionsgefäßen. Zu Beginn der Extraktion wird auch die zu bearbeitende Probe in Reaktionsgefäße überführt und auf dem Automaten platziert. Das Verfahren zur Isolierung der Nukleinsäure gliedert sich in die Prozesse Befüllung der Reaktionsgefäße, Lyse der Ausgangsprobe und Extraktion der Nukleinsäuren (via Binden der Nukleinsäuren an magnetische Partikel, Waschen der gebundenen Nukleinsäuren, Trocknung der magnetischen Partikel und Elution der Nukleinsäuren). Der gesamte erfindungsgemäße Vorgang wird dabei immer nur mit einer Reaktionseinheit pro zu bearbeitender Probe durchgeführt. Diese dient in der erfindungsgemäßen Multiplexfunktion dem Dispensieren/ Pipettieren, ggf. Filtrieren und der Extraktion der Nukleinsäure.

Die Erfindung soll nachfolgend anhand von Beispielen erläutert werden, ohne auf diese Beispiele beschränkt zu sein.

### Ausführungsbeispiele

### Beispiel 1:

Der Extraktionsprozess zur Isolierung z.B. genomischer DNA läuft wie folgt ab:

### A. Prozess Befüllung der Reaktionsgefäße

1. Aufnahme der Reaktionseinheiten durch den Dispensierkopf.
2. Befüllung der Reaktionsgefäße mit den Puffern aus den Vorratsgefäßen.

Dabei fungiert die Reaktionseinheit als klassische Pipettiervorrichtung (Puffer aufsaugen und Puffer abgeben). Die Befüllung erfolgt nach einem vorgegebenen Schema in der Art, dass immer von der Lösung mit der geringsten Salzkonzentration ausgegangen wird, so dass für die Befüllung der Reaktionsgefäße auch nur mit einer Reaktionseinheit gearbeitet wird. Befüllt wird nach folgender Reihenfolge: Elutionsmittel- Waschpuffer- Lysepuffer.

Diese Pufferkomponenten befinden sich alle in den Vorratsgefäßen auf der Automatenplattform.
3. Überführen der Probe in das Reaktionsgefäß mit vorgelegtem Lysepuffer.

### B. Prozess Lyse des Ausgangsmaterials

1. Die Lyse der Probe erfolgt (ggf. durch zugeschaltete Temperierung) durch mehrmaliges kontinuierliches Auf- und Abpipettieren des Lyseansatzes mittels der Reaktionseinheit.
2. Nach Lyse erfolgt das Aufnehmen des Bindungspuffers und magnetischer Partikel mittels der Reaktionseinheit und Überführen des Bindungspuffers und der magnetischen Partikel in den Lyseansatz. Der Bindungspuffer mit den magnetischen Partikeln befindet sich im Gegensatz zu den anderen Pufferkomponenten nicht in Vorratsgefäßen, sondern in separaten Reaktionsgefäßen (dies ist dadurch bedingt, dass eventuell noch Probenreste mit der Reaktionseinheit in den Bindungspuffer verschleppt werden können und dient damit der Verhinderung einer Kreuzkontamination).

### C. Nukleinsäure-Extraktion/ggf. Filtration von ungelösten Komponenten

1. Mischen des Lyse-/ Bindungspufferansatzes und der magnetischen Partikel mit der Reaktionseinheit (durch Auf- und Abpipettieren).
2. Bindung der Nukleinsäure an die magnetischen Partikel und nachfolgende Filtration ungelöster Stoffe sowie magnetische Separation. Dies erfolgt durch das Ansaugen der Probe vom unteren Teil der Reaktionseinheit über das Filtergitter in den oberen Teil der Reaktionseinheit. Durch diesen Vorgang wird die lysierte Probe (einschließlich der magnetischen Partikel) über das Filtergitter transportiert. Die lysierte Probe mit den enthaltenen magnetischen Partikeln befindet sich jetzt im oberen Teil der Reaktionseinheit. Die Magnetseparation erfolgt nachfolgend über das Einfahren eines Magnetstiftes aus dem Dispensierkopf in die Plastikhülse des oberen Teils der Reaktionseinheit. Bei diesem Vorgang werden die magnetischen Partikel durch den Magnetstift an die Plastikhülse gezogen und verbleiben an dieser Position. Die in der Probe eventuell vorhandenen ungelösten Komponenten befinden sich im unteren Teil der Reaktionseinheit. Nach der Anbindung der magnetische Partikel an die Plastikhülse wird die Probe nachfolgend vom oberen Teil der Reaktionseinheit wieder in das Ausgangsreaktionsgefäß zurückgegeben. Die magnetischen Partikel mit der gebundenen Nukleinsäure verbleiben an der Plastikhülse.
3. Waschen der an den magnetischen Partikeln gebundenen Nukleinsäure. Dabei Transfer der Dispensiereinheit mit Reaktionseinheit zur nächsten Reihe Reaktionsgefäße (mit enthaltenen Waschpuffern). Dieser Transfer kann sowohl durch eine schrittweise Bewegung des Dispensierkopfes erfolgen oder durch die schrittweise Bewegung der Reaktionsgefäße. In diesem Falle ist der Dispensierkopf unbeweglich. Nach erfolgtem kinematischen Schritt befindet sich die Reaktionseinheit über dem Reaktionsgefäß mit enthaltenem Waschpuffer. Nachfolgend taucht die Reaktionseinheit in das Reaktionsgefäß mit Waschpuffer ein und der Waschpuffer wird bis in den oberen Teil der Reaktionseinheit eingesaugt. Das Waschen der magnetischen Partikel erfolgt durch das Ausfahren des Magnetstiftes. Damit werden die magnetischen Partikel von der Plastikhülse freigegeben und durch wechselseitiges (ggf. mehrmaliges) Ansaugen und Ausstoßen des Waschpuffers vom unteren Teil der Reaktionseinheit über den Filter in den oberen Teil der Reaktionseinheit effizient im Waschpuffer gemischt (gewaschen). Der Waschschritt wird dadurch beendet, dass nach finalem Ansaugen des Waschpuffers in den oberen Teil der Reaktionseinheit nachfolgend der Magnetstift aus dem Dispensierkopf in die Plastikhülse des oberen Teils der Reaktionseinheit einfährt, die magnetischen Partikel durch den Magnetstift an die Plastikhülse gezogen werden und dort an dieser Position verbleiben. Final wird der Waschpuffer in das Reaktionsgefäß zurückgegeben. Die Waschschritte können durch die beschriebene Relativbewegung von Reaktionsgefäßen mit den enthaltenen Puffern oder durch Bewegung des Dispensierkopfes mehrmals wiederholt werden.
4. Trocknung des Filters. Dazu befindet sich die Reaktionseinheit über einem leeren Reaktionsgefäß. Die Trocknung erfolgt durch Anlegen von Druckluft (ggf. erwärmt) und das Durchströmen der Luft über die an der Plastikhülse magnetisch fixierten magnetischen Partikel.
5. Elution der gebundenen Nukleinsäure. Dazu Transfer der Dispensiereinheit mit Reaktionseinheit zur nächsten Reihe Reaktionsgefäße (mit enthaltenem Elutionspuffer) bzw. Transfer der nächsten Reihe Reaktionsgefäße zum feststehenden Dispensierkopf. Wiederum Reaktionseinheit in Reaktionsgefäß mit Elutionspuffer eintauchen und Elutionspuffer in den oberen Teil der Reaktionseinheit aufsaugen.

Die Elution der Nukleinsäuren von den magnetischen Partikeln erfolgt durch das Ausfahren des Magnetstiftes. Damit werden die magnetischen Partikel von der Plastikhülse freigegeben und durch wechselseitiges (ggf. mehrmaliges) Ansaugen und Ausstoßen des Elutionspuffers vom unteren Teil der Reaktionseinheit über den Filter in den oberen Teil der Reaktionseinheit effizient im Elutionspuffer gemischt. Der Elutionsschritt wird dadurch beendet, dass nach finalem Ansaugen des Waschpuffers in den oberen Teil der Reaktionseinheit nachfolgend der Magnetstift aus dem Dispensierkopf in die Plastikhülse des oberen Teils der Reaktionseinheit einfährt, die magnetischen Partikel durch den Magnetstift an die Plastikhülse gezogen werden und dort an dieser Position verbleiben. Final wird der Elutionspuffer (die isolierte Nukleinsäure) in das Reaktionsgefäß zurückgegeben.

Die isolierte Nukleinsäure liegt nun im letzten Reaktionsgefäß aufgereinigt vor und kann für die geplanten "downstream"- Anwendungen eingesetzt werden.

Wie dargestellt, ermöglicht das erfindungsgemäße Mittel und Verfahren auf ganz einfachem Wege mittels der Vorgänge des Ansaugens und Abgebens von Flüssigkeiten in Kombination mit der Nutzung magnetischer Partikel die Isolierung von Nukleinsäuren. Das "Walk-Away-Konzept" macht den Prozess der Extraktion extrem schnell, da bisher notwendige aufwendige Pipettierschritte komplett entfallen. Die multiplen Funktionen der erfindungsgemäßen Reaktionseinheit erlauben die komplette Befüllung aller notwendigen Reaktionsgefäße, ggf. das Abfiltrieren von ungelösten Bestandteilen nach Probenlyse sowie die Isolierung der Nukleinsäuren über die Anbindung an magnetische Partikel. Der bisher übliche große Verbrauch an Plastikmaterialien reduziert sich auf nur eine Reaktionseinheit/Probe. Die Integration des Lyseprozesses einschließlich ggf. der Entfernung von unlysierten Probenkomponenten in den automatisierten Extraktionsablauf stellt einen entscheidenden Vorteil gegenüber den beschriebenen alternativen und kommerziell verfügbaren Extraktionsgeräten dar. Darüber hinaus kann der erfindungsgemäße Automat durch die Auswahl des Dispensierkopfes für unterschiedliche Probendurchsätze eingesetzt werden. Damit ist das erfindungsgemäße Mittel nicht auf bestimmte Formate festgelegt und kann flexibel an die Anforderungen der Nutzer angepasst werden. Dieses Maß an Flexibilität in Bezug auf die zu bearbeitenden Probenanzahlen erlaubt es, den Automaten auch bei geringen Präparationszahlen effizient zu nutzen und dabei den Vorteil der Automatisierung des Prozesses voll auszuschöpfen.

Der Prozess der Isolierung der Nukleinsäuren wird in extrem kurzer Zeit realisiert. Nach erfolgter Lyse der Ausgangsprobe ist der Prozess der Nukleinsäureextraktion in wenigen Minuten komplett abgeschlossen, da über das "Walk-Away-Konzept" alle notwendigen Prozessabläufe extrem schnell ablaufen (z.B. keine Liquid-Handling Schritte mehr notwendig sind). Der extrem schnelle Prozessablauf in Kombination mit der Möglichkeit einer unterschiedlichen Bestückung des Automaten mit verschieden Dispensierköpfen zur Aufnahme einer unterschiedlich großen Anzahl an Reaktionseinheiten ermöglicht es, in einem vollautomatisierten Extraktionsprozess selbst große Probenumfänge extrem schnell und effizient zu bearbeiten. Darüber hinaus werden nur sehr wenige sog. "Consumables" benötigt.

Ein weiterer Vorteil der erfindungsgemäßen Mittel betrifft den Prozess der Nukleinsäureextraktion. Die Isolierung der Nukleinsäuren erfolgt wie schon beschrieben über die Anbindung der zu isolierenden Nukleinsäuren an magnetische Partikel. Dabei ist das erfindungsgemäße Mittel kompatibel zu allen dem Fachmann bekannten Verfahrenslösungen zur Isolierung von Nukleinsäuren. Dies betrifft sowohl die potenzielle Nutzung bisher bekannter unterschiedlicher magnetischer Partikel (Kombination von Chemie und Oberflächenfunktionalisierung der magnetischen Partikel) als auch die potenzielle Nutzung unterschiedlichster Pufferformulierungen, die eine Isolierung von Nukleinsäuren ermöglichen. Diese Möglichkeiten unterstreichen das enorm breite potenzielle Anwendungsspektrum des erfindungsgemäßen Mittels.

Die hohe integrative Komplexität des erfindungsgemäßen Mittels gestattet es letztlich, auf einfachem und schnellem Wege Nukleinsäuren aus unterschiedlichsten Ausgangsmaterialien hocheffizient zu isolieren, ohne Limitationen in Hinblick auf die Extraktionschemie.

Die beschriebenen Verfahrensabläufe bezogen sich bisher immer nur auf die Isolierung von Nukleinsäuren aus einer Probe. Darunter wird verstanden, dass sowohl DNA als auch RNA isoliert werden kann, bzw. durch die Wahl geeigneter Putrerkombinationen zum Anbinden der Nukleinsäuren an die magnetischen Partikel auch die Isolierung von DNA und RNA gleichzeitig erfolgt.

Sehr anspruchsvoll ist aber auch die separate Isolierung von DNA und RNA aus einer biologischen Probe. Dies ist für eine Reihe von Applikationen von großer Bedeutung.

Ziel der vorliegenden Erfindung bestand auch darin, automatisiert DNA und RNA aus einer Probe zu isolieren. Dies wird unter Verwendung der erfindungsgemäßen Reaktionseinheiten wie folgt realisiert. Wie schon dargestellt, kann die Reaktionseinheit auch für die chromatographische Isolierung von Nukleinsäure eingesetzt werden. Dazu wird auf dem Filtereinsatz der Reaktionseinheit ein dem Fachmann bekanntes chromatographisches Filtermaterial verbracht (z.B. Glasfaservlies) und mittels des oberen Teils der Reaktionseinheit fixiert. Das Verfahren der simultanen und separaten Isolierung von DNA und RNA aus einer Probe basiert auf folgendem Mechanismus. Das in der Reaktionseinheit fixierte chromatographische Material (Glasfaservlies) dient der selektiven Anbindung genomischer DNA, die zum Einsatz kommenden magnetischen Partikel dagegen der Anbindung der zu isolierenden RNA. Die Kombination eines Filtermaterials mit magnetischen Partikeln zur selektiven und separaten Isolierung von DNA und RNA sind bisher nicht beschrieben worden.

Die allgemeinen vorbereitenden Schritte der Beladung der Reaktionsgefäße auf der Automatenplattform erfolgen, wie schon beschrieben, mittels der erfindungsgemäßen Reaktionseinheit und deren Pipettierfunktion. Wenn alle benötigten Pufferlösungen vorgelegt sind, schließt sich der Prozess der separaten und parallelen Isolierung von DNA und RNA wie folgt an:

### Beispiel 2:

### A. Selektive Isolierung der genomischen DNA aus einer Probe über die Anbindung an das eingesetzte Filtermaterial.

### 1. Lyse des Ausgangsmaterials

Die Lyse der Probe erfolgt mittels dem Fachmann bekannten Lysepuffern auf der Basis stark denaturierender chaotroper Salze sowie weiteren Zusätzen wie DTT und Detergenzien (ggf. durch zugeschaltete Temperierung) durch mehrmaliges kontinuierliches Auf- und Abpipettieren des Lyseansatzes mittels der Reaktionseinheit. Verwendet wird eine erfindungsgemäße Reaktionseinheit, welche auf dem Filtereinsatz ein Glasfaservlies enthält.

### 2. Isolierung der genomischen DNA

Bindung der genomischen DNA an das Filtermaterial der Reaktionseinheit. Dies erfolgt durch wechselseitiges Ansaugen und Ausstoßen der Probe vom unteren Teil der Reaktionseinheit über den Filter in den oberen Teil der Reaktionseinheit. Durch diesen Vorgang wird die lysierte Probe über den Filter transportiert. Bei diesem Vorgang bindet die in der Probe enthaltene DNA selektiv an das Filtermaterial, nicht aber die RNA. Der Vorgang der Probenpassage über den Filter kann mehrmals erfolgen. Dies kann dadurch erreicht werden, dass die Bindungskonditionen des Lysepuffers nicht für das Anbinden von RNA geeignet, aber für das Anbinden von genomischer DNA hocheffizient sind. Final wird das Lysat mit der (nun nur noch enthaltenen RNA) in das Reaktionsgefäß zurückgegeben.
3. Waschen der am Filter gebundenen DNA. Dabei Transfer der Dispensiereinheit mit Reaktionseinheit zur nächsten Reihe Reaktionsgefäße (mit enthaltenen Waschpuffern). Wiederum Reaktionseinheit in Reaktionsgefäß mit Waschpuffer eintauchen und Waschpuffer durch wechselseitiges (ggf. mehrmaliges) Ansaugen und Ausstoßen des Waschpuffers vom unteren Teil der Reaktionseinheit über den Filter in den oberen Teil der Reaktionseinheit passieren. Schritt in einem weiteren Reaktionsgefäß mit Waschpuffer wiederholen. Final wird der Waschpuffer in das Reaktionsgefäß zurückgegeben.
4. Trocknung des Filters. Dazu befindet sich die Reaktionseinheit über einem leeren Reaktionsgefäß. Die Trocknung erfolgt durch Anlegen von Druckluft (ggf. erwärmt) und das Durchströmen der Luft über den Filter.
5. Elution der gebundenen DNA. Dazu Transfer der Dispensiereinheit mit Reaktionseinheit zur nächsten Reihe Reaktionsgefäße (mit enthaltenem Elutionspuffer). Wiederum Reaktionseinheit in Reaktionsgefäß mit Elutionspuffer eintauchen und Elutionspuffer durch wechselseitiges (ggf. mehrmaliges) Ansaugen und Ausstoßen vom unteren Teil der Reaktionseinheit über den Filter in den oberen Teil der Reaktionseinheit passieren.

Die isolierte DNA liegt nun aufgereinigt vor und kann für die geplanten "downstream"- Anwendungen eingesetzt werden.

### 3. Nachfolgende Isolierung der Total RNA über die Anbindung an magnetische Partikel.

Wie schon dargestellt, befindet sich die zu isolierende RNA der Ausgangsprobe im Lyseansatz (nach Entfernung der DNA). Vorbereitend wird die Reaktionseinheit mit enthaltenem Glasfaservlies verworfen. Der Automat übernimmt eine zweite Reaktionseinheit. Diese enthält aber kein Glasfaservlies. Der Prozess der Isolierung der RNA realisiert sich über die Anbindung an magnetische Partikel wie folgt.
1. Einstellung optimaler Bindungsbedingungen für RNA. Zugabe eines alkoholischen Bindungspuffers und der magnetischen Partikel in den verbliebenen Lyseansatz. Die Pipettierung erfolgt, wie schon beschrieben, mit der erfindungsgemäßen Reaktionseinheit. Mischen des Lyse-/ Bindungspufferansatzes und der magnetischen Partikel mit der Reaktionseinheit (durch Auf- und Abpipettieren).
2. Bindung der RNA an die magnetischen Partikel. Dies erfolgt durch das Ansaugen der Probe vom unteren Teil der Reaktionseinheit in den oberen Teil der Reaktionseinheit. Die lysierte Probe mit den enthaltenen magnetischen Partikeln befindet sich jetzt im oberen Teil der Reaktionseinheit. Die Magnetseparation erfolgt nachfolgend über das Einfahren eines Magnetstiftes aus dem Dispensierkopf in die Plastikhülse des oberen Teils der Reaktionseinheit. Bei diesem Vorgang werden die magnetischen Partikel durch den Magnetstift an die Plastikhülse gezogen und verbleiben an dieser Position. Nach der Anbindung der magnetischen Partikel an die Plastikhülse wird die Probe nachfolgend vom oberen Teil der Reaktionseinheit wieder in das Ausgangsreaktionsgefäß zurückgegeben. Die magnetischen Partikel mit der gebundenen Nukleinsäure verbleiben an der Plastikhülse.
3. Waschen der an den magnetischen Partikeln gebundenen RNA. Dabei Transfer der Dispensiereinheit mit Reaktionseinheit zur nächsten Reihe Reaktionsgefäße (mit enthaltenen Waschpuffern). Dieser Transfer kann sowohl durch eine schrittweise Bewegung des Dispensierkopfes erfolgen oder durch die schrittweise Bewegung der Reaktionsgefäße. In diesem Falle ist der Dispensierkopf unbeweglich. Nach erfolgtem kinematischen Schritt befindet sich die Reaktionseinheit über dem Reaktionsgefäß mit enthaltenem Waschpuffer. Nachfolgend taucht die Reaktionseinheit in das Reaktionsgefäß mit Waschpuffer ein und der Waschpuffer wird bis in den oberen Teil der Reaktionseinheit eingesaugt. Das Waschen der magnetischen Partikel erfolgt durch das Ausfahren des Magnetstiftes. Damit werden die magnetischen Partikel von der Plastikhülse freigegeben und durch wechselseitiges (ggf. mehrmaliges) Ansaugen und Ausstoßen des Waschpuffers vom unteren Teil der Reaktionseinheit in den oberen Teil der Reaktionseinheit effizient im Waschpuffer gemischt (gewaschen). Der Waschschritt wird dadurch beendet, dass nach finalem Ansaugen des Waschpuffers in den oberen Teil der Reaktionseinheit nachfolgend der Magnetstift aus dem Dispensierkopf in die Plastikhülse des oberen Teils der Reaktionseinheit einfährt, die magnetischen Partikel durch den Magnetstift an die Plastikhülse gezogen werden und dort an dieser Position verbleiben. Final wird der Waschpuffer in das Reaktionsgefäß zurückgegeben. Die Waschschritte können durch die beschriebene Relativbewegung von Reaktionsgefäßen mit den enthaltenen Puffern oder durch Bewegung des Dispensierkopfes mehrmals wiederholt werden.
4. Trocknung des Filters. Dazu befindet sich die Reaktionseinheit über einem leeren Reaktionsgefäß. Die Trocknung erfolgt durch Anlegen von Druckluft (ggf. erwärmt) und das Durchströmen der Luft über die an der Plastikhülse fixierten magnetischen Partikel.
5. Elution der gebundenen RNA. Dazu Transfer der Dispensiereinheit mit Reaktionseinheit zur nächsten Reihe Reaktionsgefäße (mit enthaltenem Elutionspuffer) bzw. Transfer der nächsten Reihe Reaktionsgefäße zum feststehenden Dispensierkopf. Wiederum Reaktionseinheit in Reaktionsgefäß mit Elutionspuffer eintauchen und Elutionspuffer in den oberen Teil der Reaktionseinheit aufsaugen.

Die Elution der RNA von den magnetischen Partikeln erfolgt durch das Ausfahren des Magnetstiftes. Damit werden die magnetischen Partikel von der Plastikhülse freigegeben und durch wechselseitiges (ggf. mehrmaliges) Ansaugen und Ausstoßen des Elutionspuffers vom unteren Teil der Reaktionseinheit über den Filter in den oberen Teil der Reaktionseinheit effizient im Elutionspuffer gemischt. Der Elutionsschritt wird dadurch beendet, dass nach finalem Ansaugen des Waschpuffers in den oberen Teil der Reaktionseinheit nachfolgend der Magnetstift aus dem Dispensierkopf in die Plastikhülse des oberen Teils der Reaktionseinheit einfährt, die magnetischen Partikel durch den Magnetstift an die Plastikhülse gezogen werden und dort an dieser Position verbleiben. Final wird der Elutionspuffer (die isolierte RNA) in das Reaktionsgefäß zurückgegeben.

Die isolierte RNA liegt nun im letzten Reaktionsgefäß aufgereinigt vor und kann für die geplanten "downstream"- Anwendungen eingesetzt werden.

Wie dargestellt, ermöglicht das erfindungsgemäße Mittel und Verfahren auf ganz einfachem Wege mittels der Vorgänge des Ansaugens und Abgebens von Flüssigkeiten in Kombination mit der Nutzung magnetischer Partikel und an sich bekannter chromatographischer Membranen die simultane und parallele Isolierung von genomischer DNA und RNA aus einer Ausgangsprobe. Ein weiterer Vorteil liegt auch darin begründet, dass durch die selektive Isolierung der DNA die isolierte RNA frei von genomischer DNA ist. Dies ist bisher immer ein Problem bei an sich bekannten Extraktionsverfahren zur Isolierung von zellulärer Total RNA.

Das "Walk-Away-Konzept" macht den Prozess der simultanen und parallelen Extraktion von DNA und RNA extrem schnell, da bisher notwendige aufwendige Pipettierschritte komplett entfallen. Die multiplen Funktionen der erfindungsgemäßen Reaktionseinheit erlauben die komplette Befüllung aller notwendigen Reaktionsgefäße sowie die Isolierung der DNA und RNA aus einer Ausgangsprobe. Der bisher übliche große Verbrauch an Plastikmaterialien reduziert sich auf nur zwei erfindungsgemäße Reaktionseinheiten/ Probe. Die Integration des Lyseprozesses in den automatisierten Extraktionsablauf stellt einen entscheidenden Vorteil gegenüber den beschriebenen alternativen und kommerziell verfügbaren Extraktionsgeräten dar. Letztlich kann über die erfindungsgemäße Kombination von zwei völlig unterschiedlichen festen Phasen, der Kombination von Filtermembran und magnetischen Partikeln in einem Extraktionsvorgang eine hocheffiziente und über die dargestellt Automation eine extrem schnelle simultane und parallele Isolierung von DNA und RNA aus einer Ausgangsprobe realisiert werden. Ein solches Verfahren ist bisher nicht verfügbar.

### Beispiel 3:

### Extraktionsautomat für die automatisierte Isolierung zellulärer Total RNA

Das Gerät wurde als Benchtopgerät entwickelt und besteht aus einer Kinematik mit vier Freiheitsgraden, einem 12-Kanal-Dosierkopf und einem manuell zu beladenden Tray.

Folgende Bewegungen werden von der Kinematik realisiert:
- Einziehen und Positionieren des Trays (11) in Y-Richtung.
- Positionieren des Dosierkopfes in Z-Richtung zum automatischen Aufnehmen der Tips (12), (13)
   o Zum Absenken der Tips(13) in die Tubes(1-5) und Reservoirs (6-10)

- Positionierung der Magnetsäulen (14).
- Positionierung der Dosierkolben (15).

Funktion Dosierkopf:
Der Dosierkopf kann 12 Multifunktionsspitzen (12), (13) automatisch aus dem Tray aufnehmen, parallel 12 Lösungen pipettieren und 12 Magnetsäulen zur Magnetseparation positionieren.

Die Funktion des Trays:
Der Tray wird manuell außerhalb des Gerätes mit Multifunktionsspitzen beladen und mit Lösungen befüllt. 12 Filtrate werden in den Tray eingestellt. (Siehe A. Vorbereitende Schritte)

### B. Automatisierter Extraktionsprozess

1. Zugabe magnetischer Partikel in das Filtrat von Schritt 6 und platzieren des Receiver Tubes an die Position 1 des Roboters
2. Automatische Beladung der Receiver Tubes auf dem Roboter mit folgenden Lösungen:
   Receiver Tube Position (5): RNAse freies Wasser aus Reservoir (9)
   Receiver Tube Position (4): leer
   Receiver Tube Position (3): Washing Solution LS (800 µl) aus Reservoir (8)
   Receiver Tube Position (2): Washing Solution HS (500µl) aus Reservoir (7)
   Receiver Tube Position (1): Binding Solution EG (500 µl) aus Reservoir (6)
3. Mischen des Filtrates mit den Magnetpartikeln und der Binding Solution EG an Position (1). Magnetseparation innerhalb der Reaktionseinheit. Bewegung der Reaktionseinheit zum Receiver Tube (2).
4. Waschen der Partikel in Washing Solution HS.
   Magnetseparation. Bewegung der Reaktionseinheit zum Receiver Tube (3).
5. Waschen der Partikel in Washing Solution LS.
   Magnetseparation. Bewegung der Reaktionseinheit zum Receiver Tube (4).
6. Trocknung der Partikel (4).
7. Bewegung der Reaktionseinheit zum Receiver Tube (5). Desorption der RNA von den Partikeln. Magnetseparation. RNA befindet sich im Receiver Tube an Position (5)

### ENDE der Extraktion

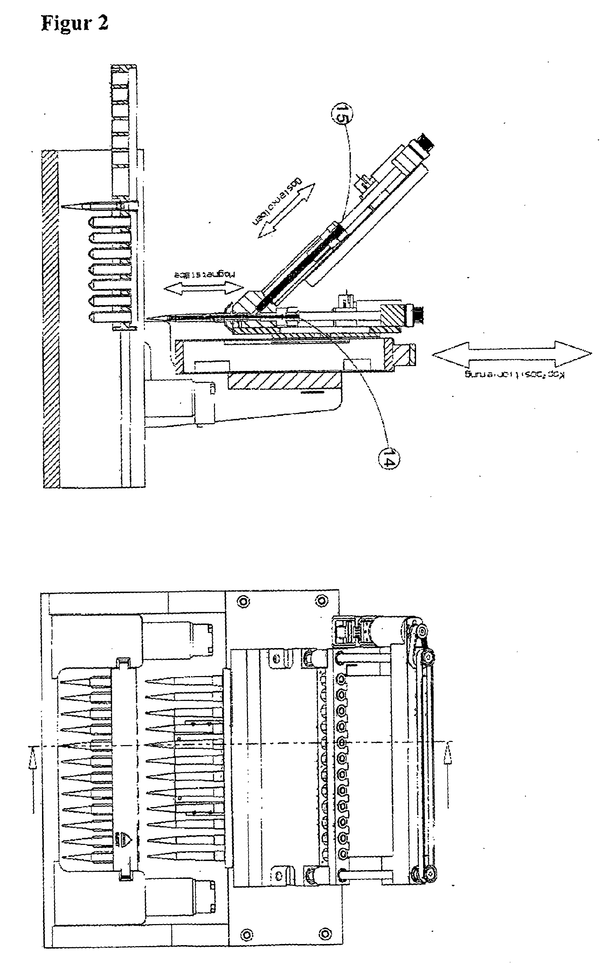

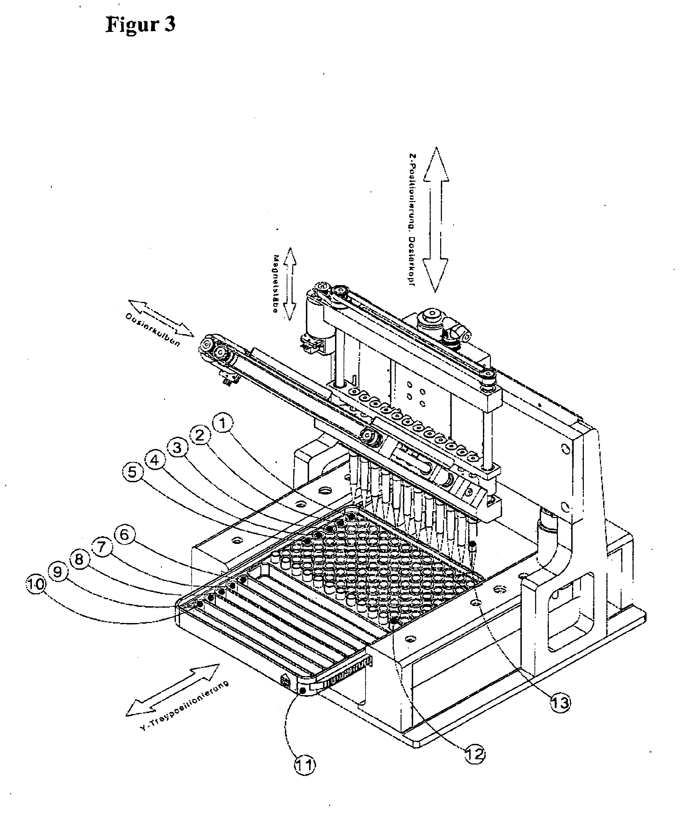

Die erfindungsgemäße Reaktionseinheit ist nachfolgend unter der Figur 1 schematisch dargestellt. Die abgebildete Variante sollen dabei keine Einschränkung des erfindungsgemäßen Mittels darstellen. Figur 2 und 3 zeigt den Extraktionsautomaten. Figur 4 zeigt eine spezielle Variante der Reaktionseinheit. Die Beschriftung in Figur 4 zeigt:

| | |
|---|---|
| 1 | Magnetpartikel |
| 2 | Magnete mit Kunststoffteilen |
| 3 | Hülse |
| 4 | Septum |
| 5 | oberer Teil der Reaktionseinheit |

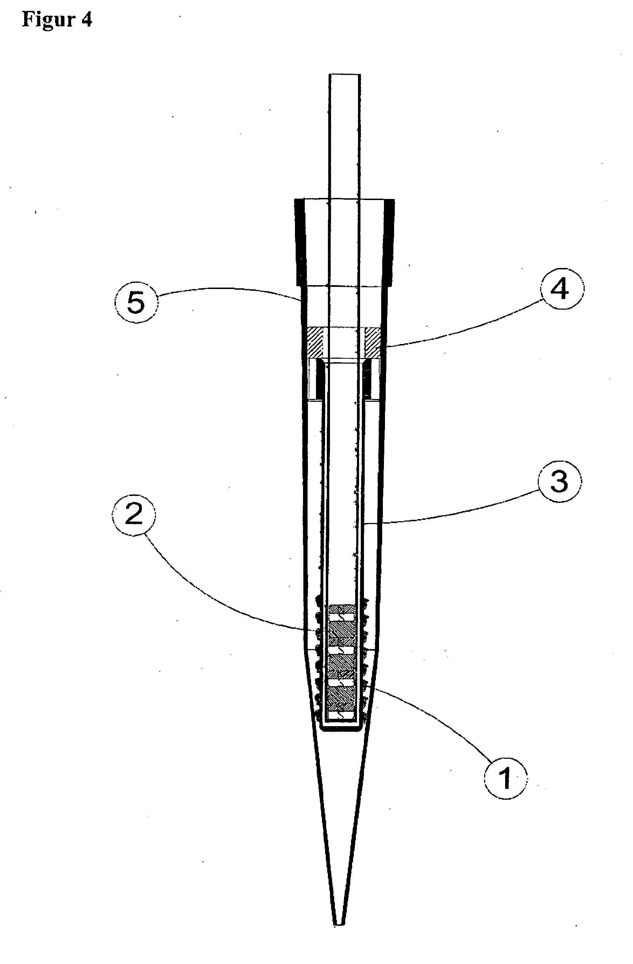

Eine durchlässiger Filtergittereinsatz ist in Figur 4 nicht abgebildet. Diese spezielle Art der Vorrichtung kann verwendet werden, wenn kein ungelösten Bestandteile abzutrennen sind. Das Septum verhindert, keine Flüssigkeit zu weit nach oben angesaugt wird.

## Patentansprüche

1. Reaktionseinheit, umfassend ein Unter- und ein Oberteil zum Pipettieren von Flüssigkeiten, **dadurch gekennzeichnet, dass** das Unterteil aus einer Reaktionskavität mit einem durchlässigen Filtergittereinsatz besteht und das Oberteil eine Reaktionskavität darstellt, die mit dem Unterteil fixiert werden kann und eine Aussparung oder eine Hülse zur Aufnahme eines Magneten enthält.

2. Reaktionseinheit nach Anspruch 1, **dadurch gekennzeichnet, dass** das Unterteil eine Pipettenspitze darstellt und das Oberteil einen Filter zum Schutz vor Aerosolen enthält.

3. Reaktionseinheit nach Anspruch 1 oder 2, **dadurch gekennzeichnet, dass** sich auf dem Filtergitter ein Trägermaterial zur Anbindung von Biomolekülen befindet.

4. Reaktionseinheit nach Anspruch 3, **dadurch gekennzeichnet, dass** als Trägermaterial ein Glasfaservlies, eine Membran oder ein chromatographisches Filtermaterial verwendet wird.

5. Reaktionseinheit nach Anspruch 3 oder 4, **dadurch gekennzeichnet, dass** das Trägermaterial durch das Aufsetzen des Oberteils auf das Unterteil an den Filtergittereinsatz fixiert wird.

6. Automat, der mindestens eine Reaktionseinheit gemäß einem der Ansprüche 1 bis 5 enthält.

7. Automat nach Anspruch 6, **dadurch gekennzeichnet, dass** es sich um einen Extraktionsautomaten handelt.

8. Automat nach Anspruch 6, **dadurch gekennzeichnet, dass** es sich um ein Benchtopgerät handelt, das eine Kinematik mit vier Freiheitsgraden enthält, außerdem einen vorzugsweise 12-Kanal-Dosierkopf und einen manuell zu beladenden Tray enthält sowie zusätzlich eine Saug- bzw. Druckluftvorrichtung enthält.

9. Extraktionsautomat nach Anspruch 8, **dadurch gekennzeichnet, dass** die Saug- bzw. Druckluftvorrichtung mit einem Dispensierkopf gekoppelt ist, der exakt auf die Reaktionseinheit gemäß einem der Ansprüche 1 bis 5 angepasst ist.

10. Extraktionsautomat nach Anspruch 8 oder 9, **dadurch gekennzeichnet, dass** die Drucklufteinheit eine Wärmequelle enthält.

11. Extraktionsautomat nach Anspruch 8, **dadurch gekennzeichnet, dass** sich im Dispensierkopf eine kinematische Einheit zur Vertikalbewegung von Magneten befindet.

12. Extraktionsautomat nach einem der Ansprüche 7 bis 11, **dadurch gekennzeichnet, dass** er eine Schnittstelle für die Kommunikation mit einem Rechner, welcher die Prozesse steuert, enthält.

13. Verfahren zur Isolierung und Aufreinigung von Nukleinsäuren aus beliebigen komplexen Ausgangsmaterialien unter Verwendung der Reaktionseinheit gemäß einem der Ansprüche 1 bis 5 und / oder des Extraktionsautomaten gemäß einem der Ansprüche 7 bis 12, **gekennzeichnet durch** folgende Schritte:
- Lyse des Ausgangsmaterials
- Binden der Nukleinsäuren an magnetische Partikel und ggf. Filtration von ungelösten Komponenten
- Waschen der gebundenen Nukleinsäuren / Trocknen
- Eluieren der gebundenen Nukleinsäuren.

14. Verfahren nach Anspruch 13, **dadurch gekennzeichnet, dass** vor der Lyse des Ausgangsmaterials Reaktionsgefäße mit Puffern aus Vorratsgefäßen unter Verwendung der Reaktionseinheit gemäß einem der Ansprüche 1 bis 5 befüllt werden.

15. Verfahren nach Anspruch 14, **dadurch gekennzeichnet, dass** die Befüllung in der Art erfolgt, dass immer von einer Lösung mit der geringsten Salzkonzentration ausgegangen wird.

16. Verfahren nach Anspruch 15, **dadurch gekennzeichnet, dass** die Befüllung in der Reihenfolge Elutionsmittel - Waschpuffer - Lysepuffer erfolgt.

17. Verfahren nach Anspruch 13, **dadurch gekennzeichnet, dass** die Lyse der Probe durch mehrmaliges kontinuierliches Auf- und Abpipettieren des Lyseansatzes mittels der Reaktionseinheit erfolgt.

18. Verfahren nach Anspruch 13, **dadurch gekennzeichnet, dass** nach der Lyse das Aufnehmen des Bindungspuffers und magnetischer Partikel mittels der Reaktionseinheit und Überführen des Bindungspuffers und der magnetischen Partikel in den Lyseansatz erfolgt.

19. Verfahren nach Anspruch 13, **dadurch gekennzeichnet, dass** der Lyse-/Bindungspufferansatz und die magnetischen Partikel mittels der Reaktionseinheit durch Auf- und Abpipettieren gemischt werden.

20. Verfahren nach Anspruch 13, **dadurch gekennzeichnet, dass** die Bindung der Nukleinsäure an die magnetischen Partikel und nachfolgende Filtration ungelöster Stoffe sowie magnetische Separation durch folgende Schritte erfolgt:
- -Ansaugen der Probe vom unteren Teil der Reaktionseinheit über das Filtergitter in den oberen Teil der Reaktionseinheit
- - Einfahren eines Magneten aus dem Dispensierkopf in die Hülse bzw. Aussparung des oberen Teils der Reaktionseinheit
- - Rückgabe der Probenflüssigkeit (ohne die an die magnetischen Partikel gebundenen Nukleinsäuren) in ein Ausgangsgefäß.

21. Verfahren nach Anspruch 13, **dadurch gekennzeichnet, dass** das Waschen der an die magnetischen Partikeln gebundenen Nukleinsäure durch folgende Schritte erfolgt:
- - Transfer der Reaktionseinheit zur nächsten Reihe Reaktionsgefäße (mit enthaltenen Waschpuffern)
- - Eintauchen der Reaktionseinheit in das Reaktionsgefäß mit Waschpuffer und Einsaugen der Waschpuffer wird bis in den oberen Teil der Reaktionseinheit
- - Entfernen des Magneten
- - mehrfaches Wiederholen der letzten beiden Schritte.

22. Verfahren nach Anspruch 21, **dadurch gekennzeichnet, dass** der Transfer sowohl durch eine schrittweise Bewegung des Dispensierkopfes erfolgen oder durch die schrittweise Bewegung der Reaktionsgefäße kann.

23. Verfahren nach Anspruch 13, **dadurch gekennzeichnet, dass** das Trocknen der an die magnetischen Partikeln gebundenen Nukleinsäure durch folgende Schritte erfolgt:
- - Transfer der Reaktionseinheit zu einem leeren Reaktionsgefäß
- - Anlegen von Druckluft / ggf. Erwärmen
- - Durchströmen der Luft über die an der Hülse bzw. Aussparung magnetisch fixierten Partikel.

24. Verfahren nach Anspruch 13, **dadurch gekennzeichnet, dass** die Elution der gebundenen Nukleinsäure durch folgende Schritte erfolgt:
- - Transfer der Dispensiereinheit mit Reaktionseinheit zur nächsten Reihe Reaktionsgefäße (mit enthaltenem Elutionspuffer) bzw. Transfer der nächsten Reihe Reaktionsgefäße zum feststehenden Dispensierkopf
- - wiederum Reaktionseinheit in Reaktionsgefäß mit Elutionspuffer eintauchen und Elutionspuffer in den oberen Teil der Reaktionseinheit aufsaugen
- - Ausfahren des Magneten
- - wechselseitiges (ggf. mehrmaliges) Ansaugen und Ausstoßen des Elutionspuffers
- - Wiederholen der letzten beiden Schritte.

25. Verfahren zur selektiven simultanen Isolierung von genomischer DNA von zellulärer RNA aus beliebigen komplexen Ausgangsmaterialien unter Verwendung der Reaktionseinheit gemäß einem der Ansprüche 1 bis 5 und / oder Extraktionsautomaten gemäß einem der Ansprüche 7 bis 12 **gekennzeichnet durch** folgende Schritte:
- - Lyse des Ausgangsmaterials
- - Isolierung der genomischen DNA
- - Isolierung der RNA.

26. Verfahren nach Anspruch 25, **dadurch gekennzeichnet, dass** die Lyse des Ausgangsmaterials durch mehrmaliges kontinuierliches Auf- und Abpipettieren des Lyseansatzes mittels der Reaktionseinheit erfolgt und sich auf dem Filtereinsatz ein Glasfaservlies oder eine Membran oder ein chromatographisches Filtermaterial befindet.

27. Verfahren nach Anspruch 25, **dadurch gekennzeichnet, dass** die Isolierung der genomischen DNA durch folgende Schritte erfolgt:
- - Bindung der genomischen DNA an das Filtermaterial der Reaktionseinheit durch mehrmaliges wechselseitiges Ansaugen und Ausstoßen der Probe vom unteren Teil der Reaktionseinheit über den Filter in den oberen Teil der Reaktionseinheit
- - Rückgabe des Lysats mit der nun nur noch enthaltenen RNA in das Reaktionsgefäß
- - Waschen der am Filter gebundenen DNA, wobei der Transfer der Reaktionseinheit zur nächsten Reihe Reaktionsgefäße (mit enthaltenen Waschpuffern) erfolgt
- - Wiederum Reaktionseinheit in Reaktionsgefäß mit Waschpuffer eintauchen und Waschpuffer durch wechselseitiges (ggf. mehrmaliges) Ansaugen und Ausstoßen des Waschpuffers vom unteren Teil der Reaktionseinheit über den Filter in den oberen Teil der Reaktionseinheit passieren (mehrmals)
- - Waschpuffer in das Reaktionsgefäß zurückgeben
- - Trocknung des Filters durch Anlegen von Druckluft (ggf. erwärmt), wobei sich die Reaktionseinheit über einem leeren Reaktionsgefäß befindet
- - Elution der gebundenen DNA durch Eintauchen der Reaktionseinheit in das Reaktionsgefäß mit Elutionspuffer und wechselseitiges (ggf. mehrmaliges) Ansaugen und Ausstoßen des Elutionspuffers vom unteren Teil der Reaktionseinheit über den Filter in den oberen Teil der Reaktionseinheit, wobei Transfer der Reaktionseinheit zur nächsten Reihe Reaktionsgefäße (mit enthaltenem Elutionspuffer) erfolgt.

28. Verfahren nach Anspruch 25, **dadurch gekennzeichnet, dass** die Isolierung der RNA durch folgende Schritte erfolgt:
- - Einstellung optimaler Bindungsbedingungen für RNA
- - Zugabe eines alkoholischen Bindungspuffers und der magnetischen Partikel in den verbliebenen Lyseansatz
- - Pipettierung mit der erfindungsgemäßen Reaktionseinheit, wobei das Mischen des Lyse-/ Bindungspufferansatzes und der magnetischen Partikel mit der Reaktionseinheit (durch Auf- und Abpipettieren) erfolgt
- - Bindung der RNA an die magnetischen Partikel durch Ansaugen der Probe vom unteren Teil der Reaktionseinheit in den oberen Teil der Reaktionseinheit
- - Magnetseparation durch das Einfahren eines Magneten aus dem Dispensierkopf in die Hülse bzw. Aussparung des oberen Teils der Reaktionseinheit
- - Rückgabe der Probe ohne die gebundenen RNA ins Ausgangsgefäß
- - Waschen, Trocknen und Eluieren der an den magnetischen Partikeln gebundenen RNA analog den Ansprüchen 21 bis 24.

29. Verfahren nach Anspruch 13 oder 25, **dadurch gekennzeichnet, dass** alle Verfahrensschritte nach dem "Walk-Away"-Prinzip ablaufen.

30. Verwendung der Reaktionseinheit gemäß einem der Ansprüche 1 bis 5 und / oder Extraktionsautomaten gemäß einem der Ansprüche 7 bis 12 für
a) eine exakte Dispensierung und Pipettierung von Flüssigkeiten und / oder
b) eine Abtrennung von festen ungelösten Komponenten nach Lyse einer zu bearbeitenden Probe und / oder
c) die Isolierung von Nukleinsäuren unter Verwendung magnetischer Partikel und / oder
d) die Isolierung von Nukleinsäuren über deren chromatographische Anbindung an ein festes Trägermaterial.

## Claims

1. A reaction unit comprising a bottom and a top part for pipetting liquids **characterised in that** the bottom part is composed of a reaction cavity with a permeable insertable filter grid and the top part represents a reaction cavity that can be fixed to the bottom part and is provided with a recess or jacket for accommodating a magnet.

2. A reaction unit according to claim 1, **characterised in that** the bottom part represents a pipette tip and the top part comprises a filter as a protection against aerosols.

3. A reaction unit according to claim 1 or 2, **characterised in that** a support material is located on the filter grid for bonding biomolecules.

4. A reaction unit according to claim 3, **characterised in that** a glass fibre mat, a membrane or a chromatographic filter material is used as a support material.

5. A reaction unit according to claim 3 or 4, **characterised in that** the support material is fixed to the insertable filter grid by placing the top part onto the bottom part.

6. A machine comprising at least one reaction unit according to claims 1 to 5.

7. A machine according to claim 6, **characterised in that** it is an extraction machine.

8. A machine according to claim 6, **characterised in that** it is a benchtop unit comprising kinematics with four degrees of freedom as well as a preferably 12 channel dosing head, and a tray to be loaded manually and in addition an intake air and / or a compressed air device.

9. An extraction machine according to claim 8, **characterised in that** the intake air and / or compressed air device is coupled with a dispensing head precisely adjusted to the reaction unit according to any one of claims 1 to 5.

10. An extraction machine according to claim 8 or 9, **characterised in that** the compressed air unit comprises a heat source.

11. An extraction machine according to claim 8, **characterised in that** a kinematic unit for vertical movement of magnets is located in the dispensing head.

12. An extraction machine according to any one of claims 7 to 11, **characterised in that** it comprises an interface for communication with a computer which controls the processes.

13. A method for isolation and purification of nucleic acids from any complex starting materials by using the reaction unit according to any one of claims 1 to 5 and / or the extraction machine according to any one of claims 7 to 12, **characterised by** the following steps :
- lysis of the starting material
- bonding of the nucleic acids with magnetic particles and, if necessary, filtration of undissolved components
- washing of bonded nucleic acids / drying
- eluting of bonded nucleic acids.

14. A method according to claim 13, **characterised in that** prior to lysis of the starting material, reaction vessels are filled with buffers from storage vessels by utilising the reaction unit according to any one of claims 1 to 5.

15. A method according to claim 14, **characterised in that** filling occurs in such a way that one always proceeds from a solution with the lowest salt concentration.

16. A method according to claim 15, **characterised in that** filling occurs in the sequence eluent - detergent buffer - lysis buffer.

17. A method according to claim 13, **characterised in that** the lysis of the sample occurs by repeated continuous pipetting up and down of the lysis preparation by means of the reaction unit.

18. A method according to claim 13, **characterised in that** after lysis the bonding buffer and the magnetic particles are taken up by means of the reaction unit and the bonding buffer and the magnetic particles are transferred into the lysis preparation.

19. A method according to claim 13, **characterised in that** the lysis / bonding buffer preparation and the magnetic particles are mixed by means of the reaction unit by pipetting up and down.

20. A method according to claim 13, **characterised in that** bonding of the nucleic acid with the magnetic particles and subsequent filtration of undissolved substances as well as magnetic separation is made by the following steps :
- aspiration of the sample from the bottom part of the reaction unit via the filter grid into the top part of the reaction unit
- introduction of a magnet from the dispensing head into the jacket and / or recess of the top part of the reaction unit
- return of the sample liquid (without the nucleic acids bonded with the magnetic particles) into an basic vessel.

21. A method according to claim 13, **characterised in that** the washing of the nucleic acid bonded with the magnetic particles is made by the following steps :
- transfer of the reaction unit to the next row of reaction vessels (with detergent buffers included)
- immersion of the reaction unit into the reaction vessel with detergent buffers and aspiration of the detergent buffers up to the top part of the reaction unit
- removal of the magnet
- repetition of the last two steps several times.

22. A method according to claim 21, **characterised in that** the transfer can occur both by a stepwise movement of the dispensing head or the stepwise movement of the reaction vessel.

23. A method according to claim 13, **characterised in that** drying of the nucleic acid bonded with the magnetic particles takes place by the following steps :
- transfer of the reaction unit to an empty reaction vessel
- application of compressed air / heating up, if required
- flowing through of air across the particles magnetically fixed to the jacket and / or recess.

24. A method according to claim 13, **characterised in that** the elution of the bonded nucleic acid takes place by the following steps :
- transfer of the dispensing unit with reaction unit to the next row of reaction vessels (with included elution buffer) and / or transfer of the next row of reaction vessels to the fixed dispensing head
- immersing reaction unit again into the reaction vessel with elution buffer and aspirating elution buffer into the top part of the reaction unit
- extension of the magnet
- reciprocal aspiration and ejection (if necessary, several times) of the elution buffer
- repetition of the last two steps.

25. A method for selective simultaneous isolation of genomic DNA of cellular RNA from any complex starting materials by using the reaction unit according to any one of claims 1 to 5 and / or extraction machines according to any one of claims 7 to 12, **characterised by** the following steps :
- lysis of the starting material
- isolation of genomic DNA
- isolation of RNA.

26. A method according to claim 25, **characterised in that** the lysis of the starting material takes place by repeated continuous pipetting up and down of the lysis preparation by means of the reaction unit and a glass fibre mat or a membrane or a chromatographic filter material is located on the insertable filter.

27. A method according to claim 25, **characterised in that** the isolation of the genomic DNA occurs by the following steps :
- bonding of the genomic DNA with the filter material of the reaction unit by repeated reciprocal aspiration and ejection of the sample from the bottom part of the reaction unit via the filter to the top part of the reaction unit
- return of the lysate with the now only included RNA into the reaction vessel
- washing of the DNA bonded with the filter with transfer of the reaction unit taking place to the next row of reaction vessels (with included detergent buffers)
- immersing reaction unit again into the reaction vessel with detergent buffer and passing detergent buffer by reciprocal (if necessary repeated) aspiration and ejection of the detergent buffer from the bottom part of the reaction unit via the filter into the top part of the reaction unit (several times)
- return of detergent buffer into the reaction vessel
- drying of the filter by application of compressed air (if necessary, heated) with the reaction unit being located above an empty reaction vessel
- elution of the bonded DNA by immersing the reaction unit into the reaction vessel with elution buffer and reciprocal (if necessary, repeated) aspiration and ejection of the elution buffer from the bottom part of the reaction unit via the filter into the top part of the reaction unit with transfer of the reaction unit to the next row of reaction vessels (with included elution buffer) taking place.

28. A method according to claim 25, **characterised in that** the isolation of the RNA takes place by the following steps :
- setting of optimum bonding conditions for RNA
- addition of an alcoholic bonding buffer and the magnetic particles to the remaining lysis preparation
- pipetting with the inventive reaction unit with mixing of the lysis / bonding buffer preparation and magnetic particles taking place with the reaction unit (by pipetting up and down)
- bonding of the RNA with the magnetic particles by aspiration of the sample from the bottom part of the reaction unit to the top part of the reaction unit
- magnet separation by introduction of a magnet from the dispensing head into the jacket and / or recess of the top part of the reaction unit
- return of the sample without the bonded RNA into the basic vessel
- washing, drying and eluting of the RNA bonded with the magnetic particles similarly to claims 21 to 24.

29. A method according to claim 13 or 25, **characterised in that** all process steps take place according to the *walk away* principle.

30. A utilisation of the reaction unit according to any one of claims 1 to 5 and / or the extraction machine according to any one of claims 7 to 12 for
a) a precise dispensing and pipetting of liquids and / or
b) a separation of solid undissolved components after lysis of a sample to be treated and /
or
c) isolation of nucleic acids by using magnetic particles and / or
d) isolation of nucleic acids via their chromatographic bonding with a solid support material.

## Revendications

1. Unité de réaction comportant une partie inférieure et une partie supérieure pour le pipettage des liquides **caractérisé en ce que** la partie inférieure comporte une cavité de réaction avec une grille de filtre insérable et perméable et que la partie supérieure présente une cavité de réaction pouvant être fixé avec la partie inférieure et contenant un évidement ou une gaine pour la réception d'un aimant.

2. Unité de réaction selon la revendication 1, **caractérisé en ce que** la partie inférieure représente une pointe de pipette et la partie supérieure contient un filtre pour protection contre des aérosols.

3. Unité de réaction selon la revendication 1 ou 2 **caractérisé en ce qu'**un matériau porteur se trouve sur la grille de filtre pour la liaison des biomolécules.

4. Unité de réaction selon la revendication 3, **caractérisé en ce qu'** un non-tissé en fibres de verre, une membrane ou un matériau de filtre chromatographique est utilisé en tant que matériau porteur.

5. Unité de réaction selon la revendication 3 ou 4, **caractérisé en ce que** le matériau porteur est fixé à la grille de filtre insérable en plaçant la partie supérieure sur la partie inférieure.

6. Machine automatique contenant au moins une unité de réaction selon une quelconque des revendications 1 à 5.

7. Machine automatique selon la revendication 6 **caractérisé en ce qu'**il s'agit d'une machine d'extraction.

8. Machine automatique selon la revendication 6, **caractérisé en ce qu'**il s'agit d'un système de table contenant de la cinématique avec quatre degrés de liberté, et une tête de dosage préférablement à 12 canaux ainsi qu'un tableau à charger manuellement et en outre un dispositif d'air d'aspiration et / ou d'air comprimé.

9. Machine d'extraction selon la revendication 8, **caractérisé en ce que** le dispositif d'air d'aspiration et / ou d'air comprimé est couplé avec une tête de dispense adaptée exactement à l'unité de réaction selon l'une quelconque des revendications 1 à 5.

10. Machine d'extraction selon la revendication 8 ou 9, **caractérisé en ce que** l'unité d'air comprimé contient une source thermique.

11. Machine d'extraction selon la revendication 8, **caractérisé en ce qu'**une unité cinématique pour le mouvement vertical des aimants se trouve dans la tête de dispense.

12. Machine d'extraction selon l'une quelconque des revendications 7 à 11, **caractérisé en ce qu'**elle contient une interface pour la communication avec un ordinateur contrôlant les processus.

13. Procédé pour l'isolation et la purification des acides nucléiques à partir des matériaux de base complexes de tout type en utilisant l'unité de réaction selon l'une quelconque des revendications 1 à 5 et / ou la machine d'extraction selon l'une quelconque des revendications 7 à 12, **caractérisé par** les étapes suivantes :
- lyse du matériau de base
- liaison des acides nucléiques aux particules magnétiques et le cas échéant filtration des composants non dissous
- lavage des acides nucléiques liés / séchage
- élution des acides nucléiques liés.

14. Procédé selon la revendication 13, **caractérisé en ce qu'**avant la lyse du matériau de base des récipients de réaction sont remplis avec des tampons à partir des récipients de stockage en utilisant l'unité de réaction selon l'une quelconque des revendications 1 à 5.

15. Procédé selon la revendication 14, **caractérisé en ce que** le remplissage s'effectue en sorte que l'on part toujours d'une solution avec la concentration la plus basse en sel.

16. Procédé selon la revendication 15, **caractérisé en ce que** le remplissage s'effectue dans l'ordre éluant - tampon détergent - tampon de lyse.

17. Procédé selon la revendication 13 **caractérisé en ce que** la lyse de l'échantillon s'effectue par un repipettage et un dépipettage continu réitéré de la préparation de la lyse au moyen de l'unité de réaction.

18. Procédé selon la revendication 13, **caractérisé en ce qu'**après la lyse le tampon de liaison et des particules magnétiques sont recueillis au moyen de l'unité de réaction et le tampon de liaison et les particules magnétiques sont transférés dans la préparation de lyse.

19. Procédé selon la revendication 13, **caractérisé en ce que** la préparation de lyse / de tampon de liaison et les particules magnétiques sont mélangées au moyen de l'unité de réaction par repipettage et dépipettage.

20. Procédé selon la revendication 13, **caractérisé en ce que** la liaison de l'acide nucléique aux particules magnétiques et la filtration suivante des substances non dissoutes ainsi que séparation magnétique s'éffectue par les étapes suivantes :
- aspiration de l'échantillon de la partie inférieure de l'unité de réaction à travers la grille de filtre dans la partie supérieure de l'unité de réaction
- introduction d'un aimant de la tête de dispense à la gaine et / ou l'évidement de la partie supérieure de l'unité de réaction
- retour du liquide de l'échantillon (sans les acides nucléiques liés aux particules magnétiques) dans un récipient de base.

21. Procédé selon la revendication 13, **caractérisé en ce que** le lavage de l'acide nucléique lié aux particules magnétiques a lieu par les étapes suivantes :
- transfert de l'unité de réaction à la file suivante des récipients de réaction (avec des tampons de lavage y inclus)
- immersion de l'unité de réaction dans le récipient de réaction avec des tampons de lavage et aspiration des tampons de lavage jusqu'à et dans la partie supérieure de l'unité de réaction
- élimination de l'aimant
- répétition multiple des deux dernières étapes.

22. Procédé selon la revendication 21, **caractérisé en ce que** le transfert peut s'effectuer non seulement par un mouvement progressif de la tête de dispense ou par le mouvement progressif des récipients de réaction.

23. Procédé selon la revendication 13, **caractérisé en ce que** le séchage de l'acide nucléique lié aux particules magnétiques s'effectue par les étapes suivantes :
- transfert de l'unité de réaction à un récipient vide
- application de l'air comprimé / chauffage, le cas échéant
- passage de l'air à travers les particules fixées à la gaine et / ou à l'évidement.

24. Procédé selon la revendication 13, **caractérisé en ce que** l'élution des acides nucléiques liés est effectué par les étapes suivantes :
- transfert de l'unité de dispense avec l'unité de réaction à la file suivante des récipients de réaction (avec tampon d'élution y inclu) et / ou transfert de la file suivante des récipients de réaction à la tête de dispense fixe
- immersion réitérée de l'unité de réaction dans le récipient de réaction avec tampon d'élution et aspiration du tampon d'élution à la partie supérieure de l'unité de réaction
- sortie de l'aimant
- aspiration et expulsion réciproque (plusieurs fois, le cas échéant) du tampon d'élution
- répétition des deux dernières étapes.

25. Procédé pour l'isolation sélective simultanée de l'ADN génomique de l'ARN cellulaire à partir des matériaux de base complexes de tout type en utilisant l'unité de réaction selon l'une quelconque des revendications 1 à 5 et / ou des machines d'extraction selon l'une quelconque des revendications 7 à 12 **caractérisé par** les étapes suivantes :
- lyse du matériau de base
- isolation de l'ADN génomique
- isolation de l'ARN.

26. Procédé selon la revendication 25, **caractérisé en ce que** la lyse du matériau de base s'effectue par un repipettage et dépipettage continu réitéré de la préparation de la lyse au moyen de l'unité de réaction et un non-tissé en fibres de verre ou une membrane ou un matériau de filtre chromatographique se trouve sur le filtre insérable.

27. Procédé selon la revendication 25, **caractérisé en ce que** l'isolation de l'ADN génomique s'effectue par les étapes suivantes :
- liaison de l'ADN génomique au matériau de filtre de l'unité de réaction par une aspiration et une expulsion réciproque réitérée de l'échantillon de la partie inférieure de l'unité de réaction à travers le filtre à la partie supérieure de l'unité de réaction
- retour du lysat avec l'ARN, qui est seulement inclu maintenant, au récipient de réaction
- lavage de l'ADN lié au filtre, le transfert de l'unité de réaction s'effectuant à la file suivante des récipients de réaction (avec des tampons de lavage y inclus)
- immersion réitérée de l'unité de réaction dans le récipient de réaction avec tampon de lavage et passage (plusieurs fois) du tampon de lavage par aspiration et expulsion réciproque (réitéré, le cas échéant) de tampon de lavage de la partie inférieure du récipient de réaction à travers le filtre à la partie supérieure de l'unité de réaction
- retour du tampon de lavage au récipient de réaction
- séchage du filtre par application de l'air comprimé (chauffé, le cas échéant), l'unité de réaction se trouvant au-dessus d'un récipient de réaction vide
- élution de l'ADN lié par immersion de l'unité de réaction dans le récipient de réaction avec tampon d'élution et aspiration et expulsion réciproque (réitéré, le cas échéant) du tampon d'élution de la partie inférieure à travers le filtre dans la partie supérieure de l'unité de réaction, le transfert de l'unité de réaction s'effectuant à la file suivante des récipients de réaction (avec tampon d'élution y inclu).

28. Procédé selon la revendication 25, **caractérisé en ce que** l'isolation de l'ARN s'effectue par les étapes suivantes :
- ajustement des conditions optimums de liaison pour l'ARN
- addition d'un tampon de liaison alcoolique et des particules magnétiques dans la préparation de lyse restante
- pipettage avec l'unité de réaction conforme à l'invention, le mélange de la préparation de lyse / de tampon de liaison et des particules magnétiques s'effectuant avec l'unité de réaction (par un repipettage et un dépipettage)
- liaison de l'ARN aux particules magnétiques par aspiration de l'échantillon de la partie inférieure de l'unité de réaction à la partie supérieure de l'unité de réaction
- séparation de l'aimant par l'introduction d'un aimant de la tête de dispense dans la gaine et / ou l'évidement de la partie supérieure de l'unité de réaction
- retour de l'échantillon sans l'ARN lié dans le récipient de base
- lavage, séchage et élution de l'ARN lié aux particules magnétiques analogue aux revendications 21 à 24.

29. Procédé selon la revendication 13 ou 25, **caractérisé en ce que** toutes les étapes du procédé s'effectuent selon le principe *walk-away.*

30. Utilisation de l'unité de réaction selon l'une quelconque des revendications 1 à 5 et / ou de la machine d'extraction selon l'une quelconque des revendications 7 à 12 pour
a) une dispense exacte et pipettage des liquides et / ou
b) une séparation des composants solides non dissous après lyse d'un échantillon à traiter et / ou
c) l'isolation des acides nucléiques en utilisant des particules magnétiques et / ou
d) l'isolation des acides nucléiques par leur liaison chromatographique à un matériau porteur solide.
